# EUROPEAN PATENT APPLICATION

(11) **EP 3 792 630 A1**
(43) Date of publication of application: **17.03.2021**
(21) Application number: 19197213.2
(22) Date of filing: 13.09.2019
(51) Int. Cl.: G01N 33/558, G01N 33/569

(54) **LATERAL FLOW IMMUNOASSAY DEVICE FOR DETECTION OF CANDIDA INFECTION**

(71) Applicant: GaDia SA, 1870 Monthey (CH)
(72) Inventor: DUCREST, Percevent, 1892 Lavey (CH)
(74) Representative: reuteler & cie SA

(57) **Abstract**

The present invention relates to a method for the detection of *Candida spp* infection in a sample by lateral flow immunoassay and a lateral flow immunoassay device, in particular useful for the early stage detection of *Candida spp* infections and uses thereof. Antibodies against Hwp1 are measured with a test line comprising Hwp1 protein fragment.

SEQUENCE LISTING

<110>  DUCREST, Percevent Jérémy

<120>  LATERAL FLOW IMMONUASSAY DEVICE AND USES THEREOF

<130>  P2355EP00

<160>  13

<170>  PatentIn version 3.5

<210>  1
<211>  148
<212>  PRT
<213>  Artificial sequence

<220>
<223>  synthetic fragment 40-187 from Candida albicans Hwp1

<400>  1

Ser Tyr Asp Tyr Tyr Gln Glu Pro Cys Asp Asp Tyr Pro Gln Gln Gln
1               5                   10                  15

Gln Gln Gln Glu Pro Cys Asp Tyr Pro Gln Gln Gln Gln Gln Glu Glu
            20                  25                  30

Pro Cys Asp Tyr Pro Gln Gln Gln Pro Gln Glu Pro Cys Asp Tyr Pro
        35                  40                  45

Gln Gln Pro Gln Glu Pro Cys Asp Tyr Pro Gln Gln Pro Gln Glu Pro
    50                  55                  60

Cys Asp Tyr Pro Gln Gln Pro Gln Glu Pro Cys Asp Asn Pro Pro Gln
65                  70                  75                  80

Pro Asp Val Pro Cys Asp Asn Pro Pro Gln Pro Asp Val Pro Cys Asp
                85                  90                  95

Asn Pro Pro Gln Pro Asp Val Pro Cys Asp Asn Pro Pro Gln Pro Asp
            100                 105                 110

Val Pro Cys Asp Asn Pro Pro Gln Pro Asp Gln Pro Asp Asp Asn Pro
        115                 120                 125

Pro Ile Pro Asn Ile Pro Thr Asp Trp Ile Pro Asn Ile Pro Thr Asp
    130                 135                 140

Trp Ile Pro Asp
145

<210>  2

<211>  160
<212>  PRT
<213>  Artifical sequence

<400>  2

Tyr Asp Tyr Tyr Gln Glu Pro Cys Asp Asp Tyr Pro Gln Gln Gln Gln
1               5                   10                  15

Gln Gln Glu Pro Cys Asp Tyr Pro Gln Gln Gln Gln Gln Glu Glu Pro
            20                  25                  30

Cys Asp Tyr Pro Gln Gln Gln Pro Gln Glu Pro Cys Asp Tyr Pro Gln
        35                  40                  45

Gln Pro Gln Glu Pro Cys Asp Tyr Pro Gln Gln Pro Gln Glu Pro Cys
    50                  55                  60

Asp Tyr Pro Gln Gln Pro Gln Glu Pro Cys Asp Asn Pro Pro Gln Pro
65                  70                  75                  80

Asp Val Pro Cys Asp Asn Pro Pro Gln Pro Asp Val Pro Cys Asp Asn
                85                  90                  95

Pro Pro Gln Pro Asp Val Pro Cys Asp Asn Pro Pro Gln Pro Asp Val
            100                 105                 110

Pro Cys Asp Asn Pro Pro Gln Pro Asp Gln Pro Asp Asp Asn Pro Pro
        115                 120                 125

Ile Pro Asn Ile Pro Thr Asp Trp Ile Pro Asn Ile Pro Thr Asp Trp
    130                 135                 140

Ile Pro Asp Ile Pro Glu Lys Pro Thr Thr Pro Ala Thr Thr Pro Asn
145                 150                 155                 160


<210>  3
<211>  177
<212>  PRT
<213>  Artifical sequence

<400>  3

Gly Gln Gly Glu Thr Glu Glu Ala Leu Ile Gln Lys Arg Ser Tyr Asp
1               5                   10                  15

Tyr Tyr Gln Glu Pro Cys Asp Asp Tyr Pro Gln Gln Gln Gln Gln Gln
            20                  25                  30

Glu Pro Cys Asp Tyr Pro Gln Gln Gln Gln Gln Glu Glu Pro Cys Asp
        35                  40                  45

Tyr Pro Gln Gln Gln Pro Gln Glu Pro Cys Asp Tyr Pro Gln Gln Pro
    50                  55                  60

Gln Glu Pro Cys Asp Tyr Pro Gln Gln Pro Gln Glu Pro Cys Asp Tyr
65                  70                  75                  80

Pro Gln Gln Pro Gln Glu Pro Cys Asp Asn Pro Pro Gln Pro Asp Val
                85                  90                  95

Pro Cys Asp Asn Pro Pro Gln Pro Asp Val Pro Cys Asp Asn Pro Pro
            100                 105                 110

Gln Pro Asp Val Pro Cys Asp Asn Pro Pro Gln Pro Asp Val Pro Cys
        115                 120                 125

Asp Asn Pro Pro Gln Pro Asp Gln Pro Asp Asp Asn Pro Pro Ile Pro
    130                 135                 140

Asn Ile Pro Thr Asp Trp Ile Pro Asn Ile Pro Thr Asp Trp Ile Pro
145                 150                 155                 160

Asp Ile Pro Glu Lys Pro Thr Thr Pro Ala Thr Thr Pro Asn Ile Pro
                165                 170                 175

Ala

<210>  4
<211>  440
<212>  PRT
<213>  Candida albicans

<400>  4

<210>  5
<211>  308
<212>  PRT
<213>  Candida albicans

<400>  5

Met Gln Ile Lys Phe Leu Thr Thr Leu Ala Thr Val Leu Thr Ser Val
1               5                   10                  15

Ala Ala Met Gly Asp Leu Ala Phe Asn Leu Gly Val Lys Asn Asp Asp
            20                  25                  30

Gly Thr Cys Lys Asp Val Ser Thr Phe Glu Gly Asp Leu Asp Phe Leu
        35                  40                  45

Lys Ser His Ser Lys Ile Ile Lys Thr Tyr Ala Val Ser Asp Cys Asn
    50                  55                  60

Thr Leu Gln Asn Leu Gly Pro Ala Ala Glu Ala Glu Gly Phe Gln Ile
65                  70                  75                  80

Gln Leu Gly Ile Trp Pro Asn Asp Asp Ala His Phe Glu Ala Glu Lys
                85                  90                  95

Glu Ala Leu Gln Asn Tyr Leu Pro Lys Ile Ser Val Ser Thr Ile Lys

            100                 105                 110

Ile Phe Leu Val Gly Ser Glu Ala Leu Tyr Arg Glu Asp Leu Thr Ala
        115                 120                 125

Ser Glu Leu Ala Ser Lys Ile Asn Glu Ile Lys Asp Leu Val Lys Gly
    130                 135                 140

Ile Lys Asp Lys Asn Gly Lys Ser Tyr Ser Ser Val Pro Val Gly Thr
145                 150                 155                 160

Val Asp Ser Trp Asn Val Leu Val Asp Gly Ala Ser Lys Pro Ala Ile
                165                 170                 175

Asp Ala Ala Asp Val Val Tyr Ser Asn Ser Phe Ser Tyr Trp Gln Lys
            180                 185                 190

Asn Ser Gln Ala Asn Ala Ser Tyr Ser Leu Phe Asp Asp Val Met Gln
        195                 200                 205

Ala Leu Gln Thr Leu Gln Thr Ala Lys Gly Ser Thr Asp Ile Glu Phe
    210                 215                 220

Trp Val Gly Glu Thr Gly Trp Pro Thr Asp Gly Ser Ser Tyr Gly Asp
225                 230                 235                 240

Ser Val Pro Ser Val Glu Asn Ala Ala Asp Gln Trp Gln Lys Gly Ile
                245                 250                 255

Cys Ala Leu Arg Ala Trp Gly Ile Asn Val Ala Val Tyr Glu Ala Phe
            260                 265                 270

Asp Glu Ala Trp Lys Pro Asp Thr Ser Gly Thr Ser Ser Val Glu Lys
        275                 280                 285

His Trp Gly Val Trp Gln Ser Asp Lys Thr Leu Lys Tyr Ser Ile Asp
    290                 295                 300

Cys Lys Phe Asn
305

<210> 6
<211> 359
<212> PRT
<213> Candida albicans

<400> 6

Met Ala Pro Pro Ala Val Leu Ser Lys Ser Gly Val Ile Tyr Gly Lys

1               5                   10                  15

Asp Val Lys Asp Leu Phe Asp Tyr Ala Gln Glu Lys Gly Phe Ala Ile
            20                  25                  30

Pro Ala Ile Asn Val Thr Ser Ser Ser Thr Val Val Ala Ala Leu Glu
        35                  40                  45

Ala Ala Arg Asp Asn Lys Ala Pro Ile Ile Leu Gln Thr Ser Gln Gly
    50                  55                  60

Gly Ala Ala Tyr Phe Ala Gly Lys Gly Val Asp Asn Lys Asp Gln Ala
65                  70                  75                  80

Ala Ser Ile Ala Gly Ser Ile Ala Ala Ala His Tyr Ile Arg Ala Ile
                85                  90                  95

Ala Pro Thr Tyr Gly Ile Pro Val Val Leu His Thr Asp His Cys Ala
            100                 105                 110

Lys Lys Leu Leu Pro Trp Phe Asp Gly Met Leu Lys Ala Asp Glu Glu
        115                 120                 125

Phe Phe Ala Lys Thr Gly Thr Pro Leu Phe Ser Ser His Met Leu Asp
    130                 135                 140

Leu Ser Glu Glu Thr Asp Asp Glu Asn Ile Ala Thr Cys Ala Lys Tyr
145                 150                 155                 160

Phe Glu Arg Met Ala Lys Met Gly Gln Trp Leu Glu Met Glu Ile Gly
                165                 170                 175

Ile Thr Gly Gly Glu Glu Asp Gly Val Asn Asn Glu His Val Glu Lys
            180                 185                 190

Asp Ala Leu Tyr Thr Ser Pro Glu Thr Val Phe Ala Val Tyr Glu Ser
        195                 200                 205

Leu His Lys Ile Ser Pro Asn Phe Ser Ile Ala Ala Ala Phe Gly Asn
    210                 215                 220

Val His Gly Val Tyr Lys Pro Gly Asn Val Gln Leu Arg Pro Glu Ile
225                 230                 235                 240

Leu Gly Asp His Gln Val Tyr Ala Lys Lys Gln Ile Gly Thr Asp Ala
                245                 250                 255

Lys His Pro Leu Tyr Leu Val Phe His Gly Gly Ser Gly Ser Thr Gln
            260                 265                 270

Glu Glu Phe Asn Thr Ala Ile Lys Asn Gly Val Val Lys Val Asn Leu
        275                 280                 285

Asp Thr Asp Cys Gln Tyr Ala Tyr Leu Thr Gly Ile Arg Asp Tyr Val
    290                 295                 300

Thr Asn Lys Ile Glu Tyr Leu Lys Ala Pro Val Gly Asn Pro Glu Gly
305                 310                 315                 320

Ala Asp Lys Pro Asn Lys Lys Tyr Phe Asp Pro Arg Val Trp Val Arg
                325                 330                 335

Glu Gly Glu Lys Thr Met Ser Lys Arg Ile Ala Glu Ala Leu Asp Ile
            340                 345                 350

Phe His Thr Lys Gly Gln Leu
        355

<210>  7
<211>  238
<212>  PRT
<213>  Artificial sequence

<220>
<223>  Synthetic fragment 1-238 from Candida albicans Pgk1

<400>  7

Met Ser Leu Ser Asn Lys Leu Ser Val Lys Asp Leu Asp Val Ala Gly
1               5                   10                  15

Lys Arg Val Phe Ile Arg Val Asp Phe Asn Val Pro Leu Asp Gly Lys
            20                  25                  30

Thr Ile Thr Asn Asn Gln Arg Ile Val Ala Ala Leu Pro Thr Ile Lys
        35                  40                  45

Tyr Val Glu Glu His Lys Pro Lys Tyr Ile Val Leu Ala Ser His Leu
    50                  55                  60

Gly Arg Pro Asn Gly Glu Arg Asn Asp Lys Tyr Ser Leu Ala Pro Val
65                  70                  75                  80

Ala Thr Glu Leu Glu Lys Leu Leu Gly Gln Lys Val Thr Phe Leu Asn
                85                  90                  95

Asp Cys Val Gly Pro Glu Val Thr Lys Ala Val Glu Asn Ala Lys Asp

            100                 105                 110

Gly Glu Ile Phe Leu Leu Glu Asn Leu Arg Tyr His Ile Glu Glu Glu
        115                 120                 125

Gly Ser Ser Lys Asp Lys Asp Gly Lys Lys Val Lys Ala Asp Pro Glu
    130                 135                 140

Ala Val Lys Lys Phe Arg Gln Glu Leu Thr Ser Leu Ala Asp Val Tyr
145                 150                 155                 160

Ile Asn Asp Ala Phe Gly Thr Ala His Arg Ala His Ser Ser Met Val
                165                 170                 175

Gly Leu Glu Val Pro Gln Arg Ala Ala Gly Phe Leu Met Ser Lys Glu
            180                 185                 190

Leu Glu Tyr Phe Ala Lys Ala Leu Glu Asn Pro Glu Arg Pro Phe Leu
        195                 200                 205

Ala Ile Leu Gly Gly Ala Lys Val Ser Asp Lys Ile Gln Leu Ile Asp
    210                 215                 220

Asn Leu Leu Asp Lys Val Asp Met Leu Ile Val Gly Gly Gly
225                 230                 235

<210> 8
<211> 208
<212> PRT
<213> Artificial sequence

<220>
<223> Synthetic fragment 1-208 from Candida albicans Set1

<400> 8

Met Ser Tyr Asn Asn Arg Ser Gly Gly Gly Ala Ser Gly Gly Tyr Ser
1               5                   10                  15

Arg Arg Gly Tyr His Gly Ser His Arg Gly Gly Tyr Arg Thr Gly Arg
            20                  25                  30

Ser Lys Tyr Pro Glu Asp Arg Tyr Leu Val Gly Gly Met Leu Ser Leu
        35                  40                  45

Asn Lys Gly Ser His Tyr Glu Ser Ser Asp Asn Arg Tyr Ile Pro Asn
    50                  55                  60

Glu Ile Gly Ser Lys Ser Pro Glu Asn Arg Ser His Arg Ser Ser Thr
65                  70                  75                  80

Lys Asp Gly Arg Thr Pro Ser Gly Leu Ser Thr Pro Leu Ser Ser Ser
                85                  90                  95

Asp Lys Val Ser Thr Pro Ile Ser Ile Glu Ser Ile Asn Gly Ser Asp
            100                 105                 110

Arg Asn Thr Gly Val Asn Asn Lys Asp Ser Glu Phe Pro Lys Leu Ser
        115                 120                 125

His His Ser Asp Phe Thr Ser Thr Ile Pro Phe Ser Arg Ser Ile Asn
    130                 135                 140

Pro Gln Lys Asn Phe Met Val Ile Asn Asp Ser His Thr Pro Lys Thr
145                 150                 155                 160

Asp Lys Gly Ile Gln Ser Lys Lys Ile Arg Tyr Asn Gly Glu Gly Val
                165                 170                 175

Asn His Val Ser Asp Pro Arg Ile Ala Gln Ser Asn Ser Asn Leu Gln
            180                 185                 190

Lys Pro Thr Lys Lys Thr Lys Lys Thr Pro Tyr Lys Gln Leu Pro Gln
        195                 200                 205

<210> 9
<211> 751
<212> PRT
<213> Candida albicans

<400> 9

Met Lys Leu Leu Pro Leu Ser Phe Ser Phe Leu Leu Phe Thr Phe Thr
1               5                   10                  15

Phe Ala Lys Gln Leu Pro Val Phe Asp Gly Lys Thr Tyr Gln Ser Tyr
            20                  25                  30

Ser His Ser Arg Asn Val Tyr Ala Cys Arg Met His Ile His Lys Thr
        35                  40                  45

Ile Lys Glu Cys Ser Thr Thr Asp Ile Glu Asp Tyr Gly Cys Met Cys
    50                  55                  60

Thr Asn Lys Asp Ala Ile Ser Thr Phe Ile Gln Cys Tyr Ala Tyr Phe
65                  70                  75                  80

Gln Lys Asn Ser Thr Gln Ala Ile Asp Phe Phe Val Lys Tyr Cys Asp
                85                  90                  95

Asn Tyr Gly Asn Ile Thr Thr Asn Ile Asp Gln Ile Thr Asp Ala Tyr
        100                 105                 110

Glu Asp Phe Leu Asn Asn Gly Ser Asp Thr Thr Thr Ile Thr Asn Ile
    115                 120                 125

Asn Asn Asn Asn Asn Thr Thr Asn Asn Thr Thr Ser Asn His Leu Asp
    130                 135                 140

Phe Asp Tyr Ser Lys Tyr Ala Tyr Gly Leu Glu Ala Ser Ile Asp Gln
145                 150                 155                 160

Phe Leu Thr Asn Trp Gln Arg Ser Phe Phe Tyr Gly Met Ala Met Ser
                165                 170                 175

Ser Tyr Trp Gly Phe Val Leu Leu Phe Gly Thr Ile Ser Asn Trp Thr
            180                 185                 190

Gln Tyr Leu Phe Pro Gly Leu Met Thr Asp Leu Thr Gly Pro Phe Ser
        195                 200                 205

Asn Trp Trp Arg Lys Asn Leu Ser Leu Pro Ala Thr Phe Gly Thr Lys
    210                 215                 220

Lys Thr Gln Glu Tyr Thr Phe Trp Phe Lys Tyr Leu Gln Gly Tyr Ile
225                 230                 235                 240

Pro Ser Arg Leu Glu Ser Ile Ile Asn Ser Gly Phe Cys Leu Val Val
                245                 250                 255

Ile Ile Val Leu Ser Ile Asn Thr Tyr Tyr Ser Val Gly Asn Glu Ile
            260                 265                 270

Ile Phe Thr Asn Tyr Val Ala Gln Leu Arg Tyr Leu Ala Asn Arg Thr
        275                 280                 285

Gly Ile Asn Ser Thr Ile Leu Met Pro Phe Leu Val Leu Phe Ala Gly
    290                 295                 300

Arg Asn Asn Ile Leu Gln Trp Ile Thr Arg Trp Asn Tyr Gly Val Phe
305                 310                 315                 320

Met Met Phe His Arg Phe Ile Gly Arg Met Ile Phe Leu Phe Val Val
                325                 330                 335

Leu His Ala Val Thr Phe Ser Ile Ala Leu Gly Val Asp Tyr Tyr Pro

            340                 345                 350

Leu Met Ser Phe Pro Phe Met Ile Trp Gly Thr Ile Ser Thr Val Ala
        355                 360                 365

Gly Gly Ile Ile Met Val Gln Ala Ser Leu Tyr Val Arg Arg Arg Trp
    370                 375                 380

Tyr Glu Val Phe Leu Gly Leu His Ile Leu Leu Ala Leu Phe Phe Met
385                 390                 395                 400

Ile Gly Gly Trp Val His Ile Ser Tyr Leu Gly Tyr Glu Trp Trp Val
                405                 410                 415

Phe Val Ala Ile Gly Leu Trp Gly Ile Asp Arg Ile Val Arg Val Leu
            420                 425                 430

Arg Met Ile Ser Phe Gly Phe Pro Glu Ala Asp Ile Thr Leu Met Ser
        435                 440                 445

Asp Glu Thr Leu Lys Ile Lys Ile Pro Arg Pro Asp Tyr Trp Glu Pro
    450                 455                 460

Ile Pro Gly Gly His Ile Phe Ile Thr Phe Leu Arg Lys Asp Cys Phe
465                 470                 475                 480

Trp Gln Ser His Pro Phe Thr Phe Ala Pro Ser Val Glu Asn Ser Asn
                485                 490                 495

Thr Ile Val Leu Tyr Thr Lys Val Lys Gln Gly Val Thr Arg Lys Leu
            500                 505                 510

Tyr Asn Tyr Leu Val Thr Cys Pro Gly Arg Arg Ala Lys Ile Thr Val
        515                 520                 525

Gly Val Glu Gly Pro Tyr Gly Glu Cys Cys Ala Ala Gly Lys Ser Gln
    530                 535                 540

Thr Ala Val Phe Ile Ala Gly Gly Asn Gly Val Pro Gly Met Phe Ser
545                 550                 555                 560

Glu Ile Tyr Asp Leu Ala Lys Arg Ser Thr Pro Asp Ser Gln Lys Arg
                565                 570                 575

Leu Lys Leu Tyr Trp Thr Ile Arg Glu Tyr Arg Ser Ile His Trp Leu
            580                 585                 590

Tyr Glu Glu Leu Asn Glu Leu Lys Lys Phe His Asn Val Glu Val Thr
        595                 600                 605

Val Phe Val Thr Lys Pro Asp Ser Asn Thr Tyr Ile Asn Glu Leu Ser
    610                 615                 620

Ser Arg Val Ile Ser Gly Lys Cys Asn Ser Thr Glu Asn Phe Asp Gly
625                 630                 635                 640

Ala Thr Thr Ala Thr Thr Ser Leu Tyr Glu Lys Ala Ser Phe Asp Asp
                645                 650                 655

Lys Ala Thr Ile Val Val Thr Asn Arg Asp Ile Thr Ser Lys Tyr Asp
            660                 665                 670

Pro Asn Ser Val Lys Asp Ala Ile Ile Ala Gln Leu Ser His Val His
        675                 680                 685

Phe Arg Glu Gly Arg Pro Lys Ile Asn Thr Ile Ile Lys Glu Glu Ile
    690                 695                 700

Glu Asp Ala Gln Gly Ser Ile Cys Phe Val Thr Cys Gly His Pro Ala
705                 710                 715                 720

Met Val Asp Asp Ile Arg Ala Glu Val Val Gly Thr Ile Gly Lys Tyr
                725                 730                 735

Lys Glu Lys Arg Ile Asp Tyr Tyr Glu Gln Leu Ile Gly Trp Ala
            740                 745                 750

<210>  10
<211>  20
<212>  PRT
<213>  Artificial sequence

<220>
<223>  Synthetic

<400>  10

Gln Glu Pro Cys Asp Tyr Pro Gln Gln Pro Gln Glu Pro Cys Asp Tyr
1               5                   10                  15

Pro Gln Gln Pro
            20

<210>  11
<211>  14
<212>  PRT
<213>  Artificial sequence

<220>
<223>  Synthetic

<400>  11

Asp Ser Arg Gly Asn Pro Thr Val Glu Val Asp Phe Thr Thr
1               5                   10


<210>  12
<211>  14
<212>  PRT
<213>  Artificial sequence

<220>
<223>  Synthetic

<400>  12

Tyr Gly Lys Asp Val Lys Asp Leu Phe Asp Tyr Ala Gln Glu
1               5                   10


<210>  13
<211>  14
<212>  PRT
<213>  Artificial sequence

<220>
<223>  Synthetic

<400>  13

Val Pro Leu Asp Gly Lys Thr Ile Thr Asn Asn Gln Arg Ile
1               5                   10

## Description

### Field of the invention

The present invention relates to a method for the detection of *Candida spp.* infection in a sample by lateral flow immunoassay and a lateral flow immunoassay device, in particular useful for the early stage detection of Candida spp. infections.

### Background of the invention

Global prevalence of health-care (acquired) infection (HCAI), also called nosocomial diseases is estimated between 7.1%- 15.5%, with an average mortality of 19-30% (Allegranzi et al., 2011, Lancet 2011; 377: 228-41). Those nosocomial infection are caused by bacteria (70-75%), yeast/fungi (up to 22%) and virus (<10%) (Weiner et al., 2016, Infection Control & Hospital Epidemiology, 37(11*)*)*. Candida spp.* is the most common fungal pathogen (Pfaller et al. 2006, Current Epidemiological Trends; Clinical Infectious Diseases, 43:S3-14). In hospital patients, colonization by *Candida spp.* occurs in up to 80% of the patients(Eggimann et al., 2011, Annals of Intensive Care, 1:37*,* Eggimann et al., 2014, Intensive Care Med, 40:1429-1448*;* Quindos et al., 2014, Rev Iberoam Micol.;31(1):42-48*).*

Candidemia (systemic blood stream infections by *Candida spp.*) is the most faced consequence (75%) of invasive candidiasis. *Candida spp.* accounts for 8-22% of all health-care -acquired bloodstream infections (BSI) and the incidence of candidemia is usually tenfold higher in intensive care units than in healthy population (Pappas et al., 2018, Nature Review-Disease Primers, 18026(4*);* Calandra et al., 2016, Critical Care, 20:125*; Eggimann et al. 2011, supra*)*.* The overall mortality attributable to candidemia is ranged from 10-47% according to the different types of studies but in some cases, mortality can be up to 70% (*Eggimann et al., 2011, supra; Pappas et al., 2018, supra*)*.*
As opposed to bacterial infections, invasive candidiasis is characterized by a 7-10-days delay between exposure to risk factors (colonization) and systemic infection development (candidemia), thus this delay is essential for an early diagnostic (*Eggimann et al*.*, 2014, supra*)*. Candida spp.* is detected on the mucosal surface of 50-70% of healthy humans. When breaches in the intestinal barriers occur, *Candida spp.* can disseminate to the abdominal cavity directly and invade the bloodstream (candidemia). Under normal conditions, *Candida spp.* behaves as a commensal organism without causing disease. However, impairment of immune response (antibiotic treatment, HIV, immunodeficiency...) can promote fungal overgrowth in the gut and candidemia, which can lead to deep-seated opportunistic infections in various organs (invasive candidiasis) (*Pappas et al.*, *2018*, *supra*)*.* Invasion of the mucosa by *Candida albicans* is monitored by the morphological changes (yeast to hypha) of cell wall (Gow et al., 2012, Nature Review-Microbiology, 10 112-122). During the yeast-to-hypha transition, genes encoding for hypha-specific proteins or *Candida albicans* germ tube protein (CAGT) such as hyphal wall protein 1 (Hwp1), hyphal regulated protein (Hyr1) and agglutinin-like sequence 3 (Als3) are upregulated (*Gow et al.*, *2012*, *supra*)*.*

To date, blood culture remains the gold standard for diagnosis of candidemia although a poor sensitivity 21-71% (*Pappas et al., 2018*, *supra;* Calandra et al., 2016, Critical Care, 20:125)*.* Furthermore, it lasts 2-4 days before *Candida albicans* is identified and proper therapeutic decisions can be made (Safavieh et al. 2017, Crit Rev Biotechnol., 37(4):441-458). Moreover, blood cultures are rarely positive in patients with deep-seated invasive candidiasis (pre-candidemia stage) and lead to undiagnosed patients and inadequate overuse of wide spectrum antibiotics (*Calandra et al., 2016, supra;* Clancy et al. 2013, Clinical Infectious Diseases, 56(9): 1284-92).

To avoid labour intensive and uncertain blood culture diagnostic methods, several biomarkers have been targeted in reason of their high concentration in the blood stream like cell wall components of *Candida spp.* such as Mannan and 1,3-β-D-glucan (BDG). These assays are based on colorimetric or turbidimetric methods. The detection of BDG is the most used in microbiological laboratories and is FDA-approved but is labour intensive, time consuming and requires highly skilled people. The low specificity of BDG and Mannan testing is due to the fact that these markers not only identify Candida species infections but other fungal infections (Angebault et al., 2016, Open Forum Infectious Diseases, 3(3), 128*; Calandra et al., 2016, supra).* The sensitivity of the test is considered as 76-95% but the specificity widely varies between 40-92% (*Pappas et al., 2018, supra*; *Angebault et al.*, *2016*, *supra*; Lamoth et al., 2012, Clinical Infectious Diseases, 54(5):633-43; Posch et al., 2017, Expert Review of Anti-infective Therapy, 15(9):829-838*;* Leon et al., 2016, Critical Care, 20:149*).*

Well-known immunogenic Candida proteins have been intensively studied as biomarker of invasive candidiasis. *Candida albicans* germ tube family (CAGT) of proteins has been targeted to develop antibodies for the diagnosis of Candidiasis (Garcia-Ruiz et al., 1997, Journal of Clinical Microbiology, 35(12*)).* More than 20 varieties of germ-tube specific antigens are known such as Hyphal wall protein 1 (Hwp1), Als3p, Ece1p, Hyr1p (Bikandi et al., 1998, Clinical and Diagnostic Laboratory Immunology, 5(3), 369-374 *;* Lain et al., 2008, Clinical and Developmental Immunology, Volume 2008, Article ID 721950, 7 pages*).* CAGT antigens are only present on the surface of the hyphal form of Candida species, being an interesting tool for the diagnostic of hyphal-mediated invasive candidiasis (*Bikandi et al., 1998, supra*)*.* Hwp1 serves as a substrate for mammalian transglutaminase, covalently linking *C. albicans'* hypha to the host cells (Mayer et al., 2013, Virulence, 4:2, 119-128*;* Staab et al. 1999, Science, 283; Nobile et al., 2006, Eukaryotic cell, 5(10) 1604-1610)*.*
Various proteins located on the cell surface of *Candida albicans* have been identified as also potential diagnostic markers. The most studied and well-documented Candida cell surface proteins is enolase (Eno) but other proteins such as fructose-bisphosphate aldolase (Fba1), phosphoglycerate kinase (Pgk1) or β-glucosidase (Bgl2) were described as potential biomarkers (Lain et al., 2007b, Clinical and Vaccine Immunology, 14(3) 318-319*;* He et al., 2015, Front. Microbiol., 6:920*;* Li et al. 2013, BMC Infectious Diseases, 13:253*; Clancy et al. 2013, supra*)*.* Lysine histone methyltransferase (Set1) and Protein similar to ferric reductase Fre10p (Cfl91) contribute to virulence and have been described as biomarkers for acute and convalescent candidemia patients with high antibody responses (Clancy et al., 2008, Journal of clinical Microbiology, 46(5) 1647-1654*;* Mochon et al., 2010, PLoS Pathog, 6(3*)).*
Diagnosis of invasive candidiasis using ELISA with Hwp1 (UniProtKB - P46593) or its N-terminal fragments (amino acids 41-200 from Hwp1 full length) has recently been reported with good sensitivity (88.9%) and specificity (90.2%) (Lain et al. 2007a, BMC Microbiology, 7:35*;* Corouge et al., 2015, PLoS ONE, 10(3*);* Mochon et al., 2010, PLoS Pathog, 6(3*);* Naglik et al., 2006, Journal of Medical Microbiology, 55 1323-1327)*.* Positive response to anti-Hwpl was detected 5 to 6 days before the isolation of *C*. *albicans* in blood (*Lain et al. 2007a, supra*)*.* WO 2014/001574 relates to methods for diagnosing invasive Candida infections based on the combined detection of "Candida glycan" (comprising Mannan, Glycan or Chitin) with known methods such as Fungitell® (Associates of Cape Cod Inc.) or Platelia™ Candida antigen Plus test (Bio-Rad Laboratories) and the presence of antibody against of a Candia protein. Among the various possible antibodies against Candida proteins to be detected in combination with Candida glycan are mentioned anti-Hwp1 antibodies and in particular for this purpose, the use in an ELISA assay of N-terminal Hwp1 polypeptides as defined in *Lain et al.*, *2017*, *supra* (fragment 41-200 of Hwp1) or a fragment 40-187 as mentioned in Fradin et al., 2008, Infection and Immunity, 4509-4517*.* WO 2014/001574 describes an improved sensitivity of a combined detection of mannan antigens (monoclonal antibody EBCA1 of Platelia™ Candida antigen Plus test) and anti-Candida protein antibodies compared to the combined detection of mannan antigens and anti-mannan antibodies. Consequently, there is a very high need to develop accurate methods and tools for early stage detection of candidemia and invasive candidiasis, i.e. before the fungal infection reaches the systemic stage in order to allow applying the adequate treatment and reduce mortality and propagation of the disease to other patients.

### Summary of the invention

The present invention relates to the unexpected finding that a lateral flow assay based on a fragment of Hwp1 (Hwp1.3) allows achieving a sensitivity higher than known assays such as those based on Mannan detection (Chumpitazi et al., 2014, Medical Mycology, 52, 460-469*;* Mikulska et al., 2010, Critical Care, 14:R222*;* Held et al., 2013, Journal of Clinical Microbiology, 51(4), 1158-1164), anti-mannan detection (*Held et al., 2013, supra; Mikulska et al., 2010, supra*) or gold-standard microbial blood analysis (*Pappas et al., 2018, supra; Calandra et al., 2016, supra; Clancy et al., 2013, supra*) and a quick response. The present invention further relates to the unexpected finding that the combination of the fragment of Hwp1 (Hwp1.3) with a cell surface protein allows the detection of both forms of the Candida infection, namely hyphal-mediated infection (invasive candidiasis) and the infection caused by non-hyphal Candida. In fact, mannan or beta-glucan are polysaccharides present on all type of cell surface of *Candida spp.* (*Gow et al., 2012, supra*) and detection of these biomarkers do not distinguish between hyphal and non-hyphal *Candida spp.* Blood culture analysis detect only the viable *Candida spp.* without distinction of the form. However, identification of the form of *Candida spp.,* responsible of the infection, can help in the management of treatment and prevention (e.g. catheter related infection, deep-seated invasive candidiasis...) and therefore the usefulness of a method of the invention are really promising.

An object of this invention is to provide a method for sensitive and specific detection of *Candida spp.* infection in a sample.

It is advantageous to provide a method for sensitive (e.g. >90%) and specific (e.g. >90%) detection of *Candida spp.* infection in whole blood, serum, plasma or bronchoalveolar lavage (BAL) samples before systemic infection stage (pre-candidemia stage).

It is advantageous to provide a method for sensitive and specific detection of *Candida spp.* infection that would allow discriminating *Candida spp.* infected samples from other fungal, bacterial or viral infections.

It is advantageous to provide a method for sensitive and specific detection of *Candida spp.* infection in bronchoalveolar lavage (BAL) samples to detect candida pneumoniae, for example for patients from intensive care unit and since BAL samples can be used for the detection of pneumoniae caused by *Candida albicans* the methods of the invention is quicker than actual microbiological analysis (Schnabel et al, 2014, Open Forum Infect Dis., 2014, 1(1*)*).

It is advantageous to provide a method for sensitive and specific detection of *Candida spp.* infection within few minutes.

It is advantageous to provide a method for sensitive and specific detection of *Candida spp.* infection, with a reduced rate of false negative (e.g. <5%).

It is advantageous to provide a method for sensitive and specific detection of invasive candidiasis (hyphal form of *Candida spp.*) and non-hyphal Candida infection.

Another object of this invention is to provide a test kit for sensitive and specific detection of *Candida spp.* infections in samples.

Objects of this invention have been achieved by providing a lateral flow immunoassay according to claim 1 or method according to claim 10.

Disclosed herein, according to a first aspect of the invention, is a lateral flow immunoassay device for qualitative or quantitative detection of Candida infection in a sample selected from a whole blood, serum, plasma and bronchoalveolar lavage (BAL) comprising a backing support and, on said backing support, a capillary flow array and a wicking pad. The capillary flow array comprises **i)** a sample receiving pad located at one end of the backing support; **ii)** a conjugate release pad being distinct from the sample receiving pad or included to the sample receiving pad and being in capillary contact with the sample receiving pad and being impregnated with at least one detection reagent comprising a first binder specific and binding to the anti-Hwpl antibody analyte conjugated to a first label moiety; **iii)** a detection pad being distinct from the conjugate release pad and being in capillary contact with the conjugate release pad and comprising a detection membrane, a capture test reagent array comprising at least one reagent test line and a capture control reagent array comprising at least one control reagent line, said capture reagent and control reagent arrays being immobilized on the detection membrane, wherein the wicking pad is located at the other end of the backing support and being in capillary contact with the detection pad and wherein said at least one reagent test line comprises a Hwp1.3 protein and said at least one control reagent line comprises a control reagent having a specific affinity for the first binder of the detection reagent. The said at least one detection reagent are diffusively releasable from the conjugate release pad. The conjugate release pad comprising a detection reagent support wherein the said at least said one detection reagent is bound in a capillary releasable manner, receives the sample (e.g. blood, serum, plasma or bronchoalveolar lavage (BAL)) from the sample receiving pad and releases the detection reagent from its detection reagent support and a first immuno-complex is formed when the anti-Hwp1 antibody analyte is present in the sample and is combined with the detection reagent. The first immuno-complex migrates to the detection pad where it binds to the said at least one reagent test line comprising the Hwp1.3 protein. The excess of detection reagent is captured by the said control reagent line.

Disclosed herein, according to another aspect of the invention, is a method for measuring the content of anti-Hwpl antibodies in a sample, said method comprising:
a) Providing a sample selected from a whole blood, plasma, serum and bronchoalveolar lavage (BAL) potentially contaminated with *Candida spp.*, diluted or not in aqueous solution;
b) Subjecting the said sample to a lateral flow immunoassay comprising **i)** a conjugate release pad comprising detection reagents for said antibodies and **ii)** a detection pad comprising a detection membrane where at least Hwp1.3 protein is bound;
c) Detecting the presence or absence of a detection line on the detection membrane at the location where the said least Hwp1.3 protein is bound, the presence of said detection line being indicative of the presence of anti-Hwp1 antibodies.

According to another aspect of the invention, is provided a kit for qualitative or quantitative detection of Candida infection in a sample, said kit comprising at least one lateral flow immunoassay device according to the invention.

### Description of the figures

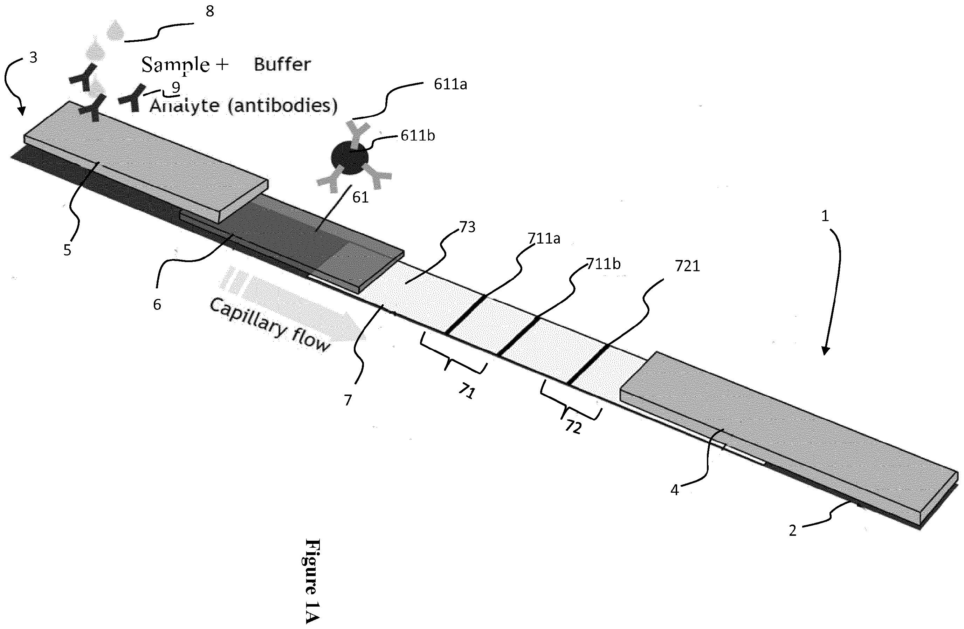

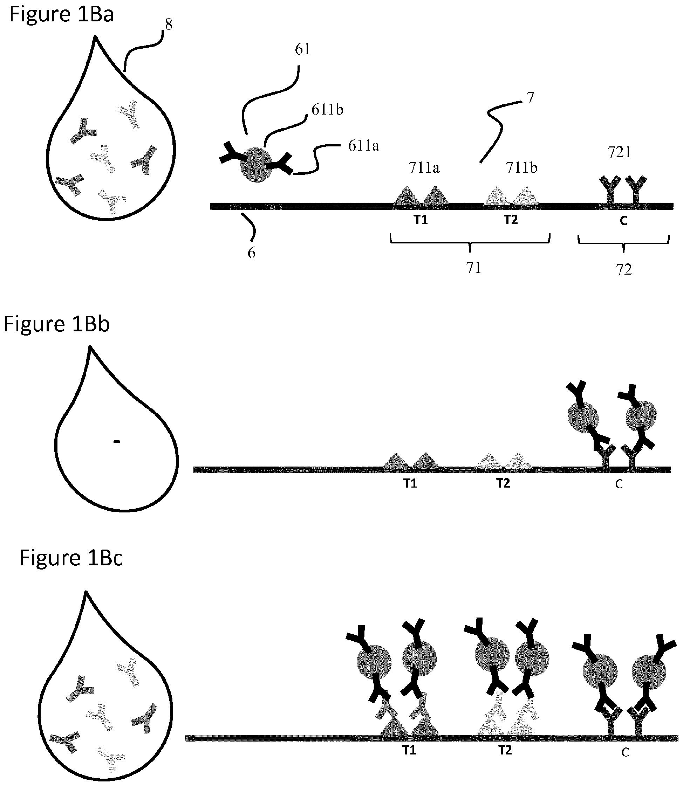

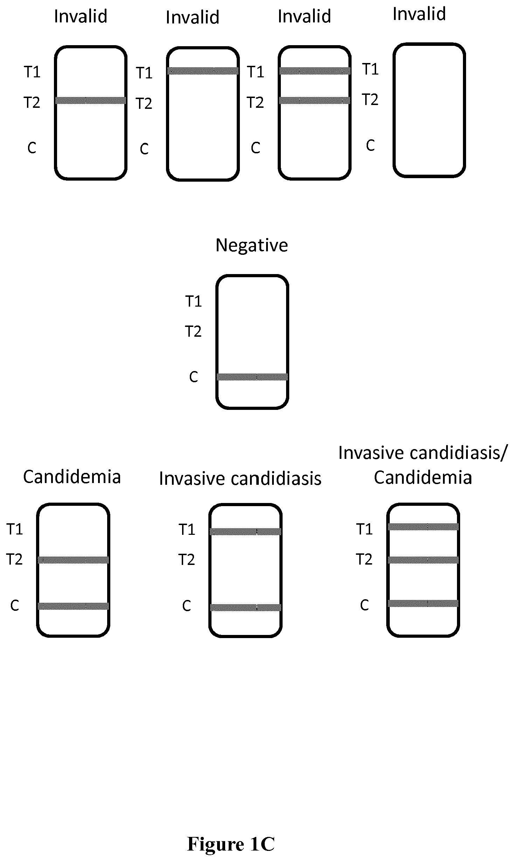

**Figure 1** is a schematic representation of a lateral flow immunoassay of the invention (**A**), the main elements used in the method (**B**) of the invention and its read outs (**C**). **B:** In a method of the invention, in particular using a lateral flow immunoassay of the invention such as described under **A**, the sample (8) containing the analyte (Human anti-Hwpl1 and/or anti-Candida protein antibodies) is subjected to the sample receiving pad and migrates to the conjugate release pad comprising detection reagents for said antibodies (first binder with first label moiety) which are then released and migrate with the sample to the detection pad comprising a detection membrane where at least Hwp1.3 protein is bound (T1: test line 1) and optionally at least one further candida protein (T2: test line 2) and a control agent specific to first binder (C: control line). In case no analyte is present (test negative), the detection reagents will only bind the control line **(****Figure 1Bb****)** and in case of the analyte is present (test positive), the analyte (human anti-Hwp1.3 and/or anti-Candida protein antibodies) will bind to specific Hwp1.3 or Candida protein on the surface of the detection pad and detection reagent (e.g. mouse anti-human IgG antibodies with first label moiety) will be attached to the analytes (human IgG antibodies fixed to test lines proteins) on the detection pad and the control line (e.g. goat anti-Mouse antibody) captures excess of detection reagent **(****Figure 1Bc****); C:** The read outs are in the form of a test line **(T)** formed by the immune-complex formed by the analyte (e.g. anti-Hwp1 or anti-Candida cell surface protein antibody), the capture test reagent (e.g. Hwp1.3 or Candida cell surface protein) and the detection reagent(s) (e.g. Gold nanoparticle conjugated with mouse anti-human IgG) and the control line (C) is formed by the immune-complex formed by the capture control reagent (e.g. anti-mouse antibody like a Goat anti-mouse antibody) and the detection reagent(s) (e.g. Gold nanoparticle conjugated with mouse anti-human IgG). Several examples of read-out are provided as an example.

### Detailed description

The term "Hwp1.3 protein" refers to a protein of SEQ ID NO: 1 and any conservative variants thereof. The term "conservative variant", applied to a peptide or polypeptide, as referred to herein means a peptide or polypeptide substantially homologous to the referenced peptide sequence, but which has at least one amino acid different from that of the referenced sequence because of one or more amino acid deletion, insertion and/or substitution. Substantially homologous means a variant amino acid sequence which is identical to the referenced peptide sequence except for the deletion and/or substitution of 1, 2, 3, 4, 5 or 6 amino acid residues. In a more particular embodiment, a variant amino acid sequence is identical to the referenced peptide sequence except for the deletion and/or conservative substitution of 1, 2, 3, 4, 5 or 6 amino acid residues. The identity of two amino acid sequences can be determined by visual inspection and/or mathematical calculation, or more easily by comparing sequence information using known computer program used for sequence comparison such as Clustal package version 1.83. A variant may comprise a sequence having at least one conservatively substituted amino acid, meaning that a given amino acid residue is replaced by a residue having similar physicochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Amino acid hydrophobicity can be found on the basis of known scales such as Kyte, et al, 1982, J. Mol. Biol., 157: 105- 131*;* Eisenberg, 1984, Ann. Rev. Biochem., 53: 595-623*.* Other such conservative substitutions, for example, substitutions of entire regions having similar hydrophobicity characteristics or rigid coiled structure of Hwp1 or substitution variants with planar secondary structure, for example assessed by circular Dichroism or Infrared spectroscopy or using any bioinformatic tools to predict protein secondary structure and rigid helical wheel properties such as Advanced Protein Secondary Structure Prediction Server (APSSP) or any other tools which are well known to the skilled persons (Kyte, et al, 1982, supra). For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in Table 1 below. The term "variant" also includes a peptide or polypeptide substantially homologous to the referenced peptide sequence, but which has an amino acid sequence different from that of the referenced sequence because one or more amino acids have been chemically modified or substituted by amino acids analogs. For example, non-natural residues can be introduced to enhance the immuno-reactivity with antibodies or binding properties (Geurink et al., 2013, J. Med. Chem., 56, 1262*;* Rand et al., 2012, Med. Chem. Commun, 3, 1282*).*

**Table 1**

| **Amino acids** | **Examples of « conservative » substitutions** |
|---|---|
| Ala (A) | Val, Leu, Ile |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, Val, Met, Ala, Phe, Norleucine |
| Leu (L) | Ile, Val, Met, Ala, Phe, Norleucine |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Trp (W) | Phe, Tyr |
| Thr (T) | Ser |
| Tyr (Y) | Trp, Phe, Thr, Ser |
| Val (V) | Ile Met, Leu, Phe, Ala, Norleucine |

The term "Hwp1.3 protein" further covers a fragment of a protein **of SEQ ID NO: 1** (Hwp1.3 fragments). According to a particular embodiment, C-term fragments of a protein of the invention have about 13 to about 25 amino acids, such as for example 14 to about 20 amino acids. For example, Hwp1.3 fragment is a peptide having a sequence QEPCDYPQQPQEPCDYPQQP (**SEQ ID NO: 10**) as described in *Corouge et al., 2015,* supra.

According to another particular embodiment, the peptides of the invention can be optionally acetylated at the N-terminus and/or amidated at the C-terminus or could be conjugated to a carrier, such as a carrier protein, for example Bovine Serum Albumin BSA or other small molecules for conjugation/binding to the surface of the detection membrane or to increase the sensibility. For example, according to a particular embodiment, when the detection membrane is a nitrocellulose membrane, peptide fragments (13 to 25 amino acids) of a protein of the invention are attached to the detection membrane through a carrier such as a carrier protein. The term "Eno protein" refers to enolase (Eno) protein (UniProtKB - P30575) as described in *Li et al. 2013, supra* and any conservative variants thereof. The term "Eno protein" further covers a fragment of a protein of **SEQ ID NO: 4** (Eno fragments). According to a particular embodiment, C-term fragments of a protein of the invention have about 13 to about 25 amino acids, such as for example 14 to about 20 amino acids. For example, Eno fragment is a peptide having a sequence DSRGNPTVEVDFTT (**SEQ ID NO: 11**) as described in Xin et al., 2008, PNAS 105(36), 13526-13531*.*

The term "Bgl2 protein" refers to β-glucosidase (Bgl2) protein (UniProtKB - Q5AMT2) as described in *He et al. 2015, supra* and any conservative variants thereof. The term "Bgl2 protein" further covers a fragment of a protein of **SEQ ID NO: 5** (Bgl2 fragments). According to a particular embodiment, C-term fragments of a protein of the invention have about 13 to about 25 amino acids, such as for example 14 to about 20 amino acids.

The term "Fba1 protein" refers to fructose-bisphosphate aldolase (Fba1) (UniProtKB - Q9URB4) as described in *Li et al. 2013, supra* and any conservative variants thereof. The term "Fba1 protein" further covers a C-term fragment of a protein of **SEQ ID NO: 6** (Fba1 fragments). According to a particular embodiment, fragments of protein of the invention have about 13 to about 25 amino acids, such as for example 14 to about 20 amino acids. For example, Fba1 fragment is a peptide having a sequence YGKDVKDLFDYAQE (**SEQ ID NO: 12**) as described in *Xin et al., 2008, supra* and in Shi et al., 2018, Vaccine, 36(38):5717-5724.

The term "Pgk1 protein" refers to phosphoglycerate kinase (Pgk1) protein (UniProtKB - P46273) (sequence of amino acid 1-238 described in Clancy et al., 2008, Journal of clinical Microbiology 2008; 46(5) 1647-1654) and any conservative variants thereof. The term "Pgk1 protein" further covers a fragment of a protein of **SEQ ID NO: 7** (Pgk1 fragments). According to a particular embodiment, C-term fragments of a protein of the invention have about 13 to about 25 amino acids, such as for example 14 to about 20 amino acids. For example, Pgk1 fragment is a peptide having a sequence VPLDGKTITNNQRI (**SEQ ID NO: 13**) as described in *Xin et al.**,** 2009, supra.*

The term "Set1 protein" refers to Lysine histone methyltransferase (Set1) protein (UniProtKB - Q5ABG1) (**SEQ ID NO: 8**) as described in *Clancy et al., 2008, supra* and any conservative variants thereof. Set1 was previously shown to contribute to virulence and to have higher antibody responses among patients with systemic candidiasis than among controls. *Clancy et al., 2018, supra* have shown that Set1 protein represent one of the best biomarkers for the diagnostic of systemic candidiasis with sensitivity of 98.3%.

The term "Cfl91 protein" refers to Protein similar to ferric reductase Fre10p (Candida Genome Database - CR_06870C_A) (**SEQ ID NO: 9**) as described in *Mochon et al., 2010, supra* and any conservative variants thereof. Cfl91 is a putative ferric reductase similar to Fre10, which is required for the release of iron from transferrin and the reduction to ferrous iron. Cfl91 is found as a biomarker for both acute and convalescent candidemia patients (*Mochon et al., 2010, supra*)*.*

The term "whole blood sample" is intended to mean any blood sample diluted or not and/or placed in aqueous solution.

The term "aqueous solution" is intended to mean containing water or placed in water e.g. purified, distilled, sterile water or water for injection.

The expression "container" refers to any recipient suitable for sample collection, sample dilution and sample preparation such as vials, centrifuge tubes, flasks, Eppendorf tubes, micro-centrifuge tubes, U-shaped tubes, blood collection tubes, thistle tubes, hybridization tubes, capillary tubes, wintrobe tubes, culture tubes, micro-titer tubes, hematocrit tubes and micro-hematocrit tubes.

### Assay of the invention

Referring to the figures, in particular first to Figure 1A, a lateral flow immunoassay device for qualitative or quantitative detection of Candida infection in a sample selected from a whole blood, plasma, serum and bronchoalveolar lavage (BAL) comprises:
- a backing support 2
- a capillary flow array 3 on said backing support,
- a wicking pad 4,
the capillary flow array 3 comprises **i)** a sample receiving pad 5 located at one end of the backing support; **ii)** a conjugate release pad 6 being distinct from the sample receiving pad or being integrated to the sample receiving pad and being in capillary contact with the sample receiving pad 5 and being impregnated with at least one detection reagent 6 comprising a first binder specific and binding to the Human IgG antibodies analyte 611a conjugated to a first label moiety 611b; **iii)** a detection pad 7 being distinct from the conjugate release pad and being in capillary contact with the conjugate release pad and comprising a detection membrane 73, a capture test reagent array 71 comprising at least one reagent test line 711a and a capture control reagent array 72 comprising one control reagent line 721, said capture reagent and control reagent arrays being immobilized on the detection membrane 73, the wicking pad 4 being at the other end of the backing support 2 and being in capillary contact with the detection pad 7, wherein said at least one reagent test line 711a comprises a Hwp1.3 protein and said control reagent line comprises a control reagent having a specific affinity for the first binder 61. Optionally, the capture test reagent array 71 may comprise further reagent test lines 711b,...n, such Eno, Fba1 etc... proteins.

The conjugate release pad 6 comprises a detection reagent support 62 wherein the said at least said one detection reagent 61 is bound in a capillary releasable manner, receives sample 8 (e.g. whole blood, plasma, serum or BAL) from the sample receiving pad 5 and releases the detection reagent 61 from its detection reagent support 62 and a first immuno-complex is formed when an antibody analyte 9 (e.g. human anti-Hwp1 IgG or other specific antibodies such as human anti-Eno, human anti-Fba1, IgG antibodies) is present in the sample and is combined with the detection reagent 61. The first immuno-complex migrates to the detection pad 7 where it binds to the said at least one reagent test line 711a or 711b, 711n (depending of the antibody present in the sample) from the capture reagent array 71 comprising the Hwp1.3 protein. The excess of detection reagent 61 is captured by the said one control reagent line 721a from the capture control reagent array 72.

According to a particular embodiment, the backing support is made of a non-porous material such as polyvinyl chloride (PVC).

According to another particular embodiment, the sample receiving pad has pores so as to receive the whole blood, plasma, serum or BAL sample. Typically, the sample receiving pad comprises an absorbant pad on to which the sample is applied and is typically composed of a woven mesh or cellulose filter. Irrespective of which material is chosen, the sample receiving pad should exhibit consistent absorbency, thickness and density so that uniform wicking rates ensure assay reproducibility. The sample receiving pad should also have low protein binding to avoid loss of analyte. Pre-treatment such as blocking with protein (e.g. BSA, milk protein, gelatine, ...) or polymer/surfactant could be applied to the sample receiving pad to reduce non-specific binding of the analyte. The sample receiving pad could be designed in such way to have red blood cells retention properties.

According to another particular embodiment, the conjugate pad could be made of the same material/support than the sample receiving pad.

According to another particular embodiment, the detection reagent support of the conjugate release pad is typically composed of non-woven glass fiber into which the detection reagent has dried. Once the sample receiving pad has been saturated, the sample flows in to the conjugate release pad where it releases the detection reagent(s) which in turn leave(s) the conjugate release pad and moves with the sample in the detection reagent support to the detection membrane of the detection pad.

According to another particular embodiment, the detection reagent is specific to human IgG (e.g. to human anti-Hwp1.3 IgG, human anti-Eno IgG, human anti-Fba1 IgG etc), such as an anti-human IgG antibody like a mouse or rabbit anti-human IgG antibody (first binder specific and binding to the antibody analyte) conjugated to colored or fluorescent particles (label moiety).

According to another particular embodiment, the detection reagent is specific to human IgA.

According to a particular aspect, the label moieties can be gold nanoparticles, latex beads, cellulose beads, fluorescent labels/particles and other colloidal metals and magnetic particles which produce a colored/fluorescent read out or any other particles generally used in lateral flow assays. According to a further embodiment, the label moiety is a gold, cellulose or latex particle.

According to a particular aspect, the detection membrane can be made a membrane of nitrocellulose membrane or cellulose acetate, polyvinylidene fluoride (PVDF), charge-modified nylon, polyethersulfone (PES) or treated/non-treated cellulose. Typically, nitrocellulose membranes exhibit a range of pore sizes (0.05 to 12 µm) and are suitable for the present assay.

According to a particular aspect, capillary flow values expressed in Capillary speed down web for purified water (s/40mm) between 90 and 180 are suitable for the present assay.

Capture reagents are immobilized across the detection membrane, typically in two capture reagent arrays (test and control reagents), respectively and each of the capture reagent arrays comprise one or more lines. The test line(s) is used to bind the sample target analyte (e.g. antibodies, while the control line consists of agents specific for the detection of the first binders such as species-specific antibodies (e.g. anti-mouse/anti-rabbit antibodies) and is used to demonstrate that the lateral flow immunoassay is performing as it should be.

According to a particular aspect, the wicking pad is composed of a cellulose/cotton fiber.

According to a particular aspect, the lateral flow immunoassay may be inserted into a rigid housing (e.g. a plastic cassette) protecting the capillary flow array and wicking pad and having an opening over the sample receiving pad for loading the sample on to said sample receiving pad and one opening over the capture reagent array for the visual interpretation of the test (presence/absence of colored lines).
According to a particular embodiment, the detection pad may comprise more than one reagent test line in the capture test reagent array. In particular, the capture test reagent array may comprise a Hwp1.3 protein and at least one Candida cell surface protein, such as for example selected from Eno, Bgl2, Fba1, Pgk1, Set1 and Cfl91.

According to a particular embodiment, the control agent specific to first binder is a species-specific antibody to the anti-human IgG or IgA antibody bound to detection particles (e.g. anti-mouse or anti-rabbit).

### Preparation of an immunoassay of the invention

A sample receiving pad can be purchased from Ahlstrom-Munksjö CytoSep® as a single layer plasma separation media consisting of high purity natural and synthetic fibers for whole blood diagnosis such as Ref. A1660, A1667 or A1668. Pre-treated or not conjugate pads can be made of glass and/or polyester fibers such as Ahlstrom-Munksjö Ref. A8964 or ReliaFlow™. According to a particular embodiment, the detection membrane can be made of nitrocellulose such as Hi-Flow™ Plus from Millipore, HF-120, HF-135 or HF-180 (preferred), representing the capillary flow time (sec/4cm). Treatment of the detection membrane with blocking proteins such as BSA, casein, gelatin, milk protein or polymers/surfactant could be realized to reduce background signal and to reduce non-specific binding of analyte. The wicking pad serves as absorbent of liquid excess and needs to have good absorbent properties. For example, cotton fiber from Ahlstrom-Munksjö Ref. A222 or A 237 can be used.
Recombinant proteins can be used as capture reagent test proteins (Hwp1.3, Eno...) for the test lines, i.e. produced in *Escherichia coli* and diluted to a concentration between 0.1 and 1 mg/ml in as PBS or other low salt buffer. Addition of blocking/carrier proteins, surfactant or alcohol (ethanol) could be used to optimize the binding and drying of test lines onto the detection membrane. Polyclonal antibodies (e.g. Goat anti-mouse antibody) with minimal cross-reaction to serum protein and other species, are diluted in PBS or other low salt buffer to a concentration between 0.1 and 1 mg/ml can be used as control agent.

Monoclonal mouse anti-human IgG antibody conjugated to detection particles can be sued as detection reagents.
All paper and membrane (sample receiving pad, conjugate pad, detection membrane and wicking pad) could be pre-treated and are cut with respective width, depending of the length of the capillary flow array and the superposition of the different membranes. If pre-treatments with blocking solution occur, treated membrane need to be dried in specific condition (such as 37°C, 10% RH (relative humidity), 2 hours or overnight at room temperature and 10% RH). Spraying of the detection reagent on the detection reagent support from the conjugate release pad can be realized with a dispensing machine (BioDot or Kinematic Automation) with air pressure (e.g. 2 PSI), pump dispense flow of e.g. 1 *µ*l/cm and bed speed of e.g. 5 cm/second.
Sprayed detection reagent support can be dried with same condition as above. Recombinant proteins (test lines from capture test reagent array) and capture control antibody (control line) are diluted and applied in different position on the detection membrane using similar dispensing machine, (e.g. with pump dispense flow of 1 *µ*l/cm and bed speed of 5 cm/second). Detection membrane is dried under same condition cited above. Assembling of all components of the assay is made by hand or with an automatized process. Assembled cards with different paper and membrane could be covered with a cover tape (transparent adhesive plastic) in order to protect the assay integrity. Assembled cards are then cut in strip suitable for the assay (e.g. with a width between 3.0 and 7.0 mm). Cut strips can then be assembled in a plastic cassette in order to protect the strip, pouched with a desiccant in an aluminum pouch and sealed with a thermo-sealing machine. All production and assembling steps take place in clean room with controlled temperature (<25°C) and humidity (<40%RH).

Assembling of the final kit with accessories (optional), test in sealed pouch, instruction for use in a kit box can be done in a non-controlled environment.

### Methods of the invention

Disclosed herein, according to a second aspect of the invention, is a method for measuring the content of anti-Hwpl antibodies in a sample, said method comprising:
a) Providing a sample selected from a whole blood, serum, plasma and bronchoalveolar lavage (BAL) potentially contaminated with Candida spp., optionally diluted in aqueous solution;
b) Subjecting the said sample to a lateral flow immunoassay comprising **i)** a conjugate release pad comprising detection reagents for said antibodies and **ii)** a detection pad comprising a detection membrane where at least Hwp1.3 protein is bound;
c) Detecting the presence or absence of a detection line on the detection membrane at the location where the said least Hwp1.3 protein is bound, the presence of said detection line being indicative of the presence of anti-Hwpl antibodies.

According to a particular aspect, the detection of the presence of anti-Hwpl antibodies according to a method of this invention or by an immunoassay of the invention is indicative of hyphal-mediated Candida infection, i.e. invasive candidiasis.

According to another further particular aspect, is provided a method according to the invention wherein at least one Candida cell surface protein, for example selected from Eno, Bgl2, Fba1, Pgk1, Set1 and Cfl91 or a combination of those, is further bound to the detection membrane of said lateral flow immunoassay and the detection of the presence of antibodies to this said at least one Candida cell surface protein is further carried out under step c). The detection of the presence of anti-Candida cell surface protein antibodies according to a method of this invention or by an immunoassay of the invention is indicative of non-hyphal-mediated Candida infection.

According to another further particular aspect, is provided a method according to the invention wherein at least one Candida cell surface protein is Eno.
According to another further particular aspect, is provided a method according to the invention wherein at least one Candida cell surface protein is Bgl2.

According to another further particular aspect, is provided a method according to the invention wherein at least one Candida cell surface protein is Fba1.
According to another further particular aspect, is provided a method according to the invention wherein at least one Candida cell surface protein is Pgk1.
According to another further particular aspect, is provided a method according to the invention wherein at least one Candida cell surface protein is Set1.
According to another further particular aspect, is provided a method according to the invention wherein at least one Candida cell surface protein is Cfl91.

### Kits of the invention

According to a particular aspect of the invention, the said at least one lateral flow immunoassay device is sealed in a pouch with a desiccant.
According to a particular aspect of the invention, the said at least one lateral flow immunoassay device is assembled in a transparent plastic cassette.
According to a particular aspect of the invention is provided a kit of the invention may further comprise at least one of the following:
- a sample collection device (e.g. pipette or automatic lancet for finger prick blood sample or capillary tube, capillary pipette);
- a container with a buffering solution for dilution of the sample or for running the test with pure sample (e.g. Low-density polyethylene (LDPE) plastic bottle with cap and dropper or any other closed plastic bottle with dropper);
- instructions for use.

According to a particular aspect of the invention is provided a kit of the invention may optionally further comprise or more of the following:
- a disinfection pad;
- an adhesive plaster;
- a data reader for reading the assay results and optionally transmitting to a database (e.g. patient hospitalization data set).

According to a further aspect, is provided a kit according to the invention for conducting a method of the invention and its use in a method according to the invention.

According to a further aspect, an assay or a kit according to the invention are useful for detection of hyphal-mediated infection (invasive candidiasis).

According to a further aspect, an assay or a kit according to the invention are useful for detection of both forms of the Candida infection, namely hyphal-mediated infection (invasive candidiasis) and Candida infection caused by non-hyphal Candida.

According to a particular embodiment, the method and assay according to the invention allow the detection of a Candida infection in a very rapid manner (less than 30 minutes) at a very early stage of the infection (i.e. before the systemic bloodstream infection stage) with a high selectivity and high sensitivity (e.g. >90%).

Examples illustrating the invention will be described hereinafter in a more detailed manner and by reference to the embodiments represented in the Figures.

### EXAMPLES

The following abbreviations refer respectively to the definitions below:
**BSA** (bovine serum albumin); **PBS** (Phosphate-Buffered Saline); **TBS** (Tris-Buffered Saline) **TBST** (Tris-Buffered Saline with Tween).

### Example 1: Preparation of a lateral flow assay of the invention and comparison of performance

A lateral flow immunoassay of the invention was prepared as follows:

### Production of the "half-sticks" containing only the detection and wicking pads on the backing support

The production of half-sticks for the liquid phase of the test has been performed in a clean, controlled room (EU GMP Class D), temperature (<25°C) and humidity (<40% RH). A nitrocellulose membrane (width 25 mm) HF-180 (Merck Millipore) was used as detection membrane. Bands of absorbent pad A222 (Ahlstrom-Munksjö) with a width of 21 mm were cut and paste on the upper part of the backing support and used as wicking pad. Strips were cut with a width of 5 mm (± 0.5 mm) and stored in aluminum pouches with desiccants at room temperature.

### Spotting of biomarkers on the detection membrane of the half-sticks

Several biomarkers were used as test reagents: the biomarkers were each diluted at a specific concentration, indicated in Table 1 below, in TBS 1X. Then, 0.5µL of each diluted biomarker were spotted on the nitrocellulose membrane (detection membrane) and let dry at 25°C, <40%RH for 2 hours to form a test reagent line. Hwp1.3 corresponds to a test protein of the invention which sequence (**SEQ ID NO: 1**) corresponds to amino acids 40-187 of full length Hwp1. Other fragments of full length Hwp1, namely fragment 41-200, Hwp1.2 (**SEQ ID NO: 2**) used in the ELISA and immunoblotting assay of *Lain et al., 2017, supra* and fragment 27-203 Hwp1.1 (**SEQ ID NO: 3**) WO 2014/001574 and fragment 27-203 described in *Fradin et al., 2008, supra* were used as comparatives.

**Table 1**

| **Test reagents** | **Sequence** | **Supplier ref.** | **Concentration [mg/ml]** |
|---|---|---|---|
| Hwp1.3 | SEQ ID NO: 1 | Genscript | 0.16 |
| Hwp1.2 (comparative) | SEQ ID NO: 2 | Genscript | 0.11 |
| Hwp1.1 (comparative) | SEQ ID NO: 3 | Cusabio, CSB-YP343212CZD | 0.4 |
| Candida Eno | SEQ ID NO: 4 | Diarect, 45301 | 0.6 |
| Candida Bgl2 | SEQ ID NO: 5 | Diarect, 44801 | 0.5 |
| Candida Fba1 | SEQ ID NO :6 | MyBioSource MBS1345647 | 1.0 |

### Test with clinical samples

Positive samples serum (n=6) of patients with Candida sp. in blood were purchased from Discovery Life Sciences (Huntsville, USA) or were selected in the sera-bank from Augurix SA (Monthey, Switzerland). These samples were preliminary selected on the basis of the presence of *Candida albicans* in blood or suspected infection with Candida sp. Negative samples (n=8) were selected from the sera-bank of Augurix SA. All samples (n=14) were sent to Unilabs Switzerland (Coppet, Switzerland) for characterization using mannan and anti-mannan test (ELISA Fungitell™ and Platelia™).

Each Samples (25 µL) were mixed with 6 µL of 40 nm-Gold nanoparticles anti-human IgG (NanoImmunoTech, Zaragoza, Spain) used as detection agents and 2 µL of Tween 20 (Sigma-Aldrich, Darmstadt, Germany) on 96-well plate. Detection membranes (cellulose half-sticks prepared as described above) were added onto each well and the solution migrated during 5 minutes on the strip. Strips were visually read and results were expressed as positive (presence of candidemia) or negative (absence of candidemia) depending on the color of the spot: If a red line appears, it means a positive test and no red line means a negative test (see Figure 1C). Threshold is fixed at Rann score 4 standardly used for lateral flow assays to define visually the intensity of a colored line (the scale goes from 0 (no line) to 10 (line with high intensity).

All sera were preliminary characterized using available data such as microbial analysis (*C*. *albicans*), disease state (celiac positive, lactose intolerance) and in order to confirm the first characterization, samples were sent to Unilabs for a re-characterization using mannan/anti-mannan tests based on ELISA (in order to have comparative analysis).

Based on these results, diagnostic performances of each biomarker were calculated separately, by excluding samples where characterization not available for a sample size of n=14. The sensibility, specificity, positive predictive value (PPV) and negative predictive value (NPV) were calculated as described in Parikh et al., 2008, Indian J Ophthalmol., 56(1): 45-50 and listed in Table 2 below.

**Table 2**

| **Test reagents** | **Sensibility %** | **Specificity %** | **PPV %** | **NPV %** |
|---|---|---|---|---|
| **Hwp1.3** | **83.3** | **75** | **71.4** | **85.7** |
| Candida Eno | 66.7 | 100 | 100 | 80 |
| Candida Bgl2 | 66.7 | 100 | 100 | 80 |
| Candida Fba1 | 66.7 | 100 | 100 | 80 |
| Hwp1.1 | 66.7 | 87.5 | 80 | 77.8 |
| Hwp1.2 | 33.3 | 100 | 100 | 66.7 |

Diagnostic performances are clearly above the average for the test proteins Eno, Bgl2, Fbal and Hwp1.3. Combination of Hwp1.3 with Eno, Bgl2 or Fba1 give always diagnostic performances above the average. Comparative fragment Hwp1.2, used in *Lain et al, 2007, supra* shows low sensitivity. This fragment was reported to present a low sensitivity (27.8%) by immunoblotting and high sensitivity (88.9%) by ELISA.

The best sensitivity of the later flow assay was obtained with Hwp1.3 used as test protein and the assay has similar performances than the reported performances for ELISA described in *Lain et al., 2017, supra.*

### Control with specific antibodies

In order to control the specificity of the test, specific antibody anti-Hwp1 (mAb 16B1, Lot.051118, as described in Marot-Leblond et al., 2000, Journal of clinical Microbiology, 38(1), 61-67) (KaliDiv, SARL, Arvillé, France) was serial diluted (dilution following a logarithmic scale) in TBST + 1% BSA containing goat anti-mouse IgG conjugated to cellulose nanoparticles (Asahi Kasei, Japan).

Detection membranes containing Hwp1.3 or the control fragments Hwp1.1 or Hwp1.2 (nitrocellulose half-sticks prepared as described above) used as test reagent lines (concentration Table 1) were added onto each well and the solution migrated during 10 minutes on the strip. The limit of detection (LOD), representing the lowest dilution where the signal is still positive, was determined for each Hwp1 fragment.

The reactivity and specificity of all fragment of Hwp1 was confirmed with the specific anti-Hwp1 monoclonal antibody and the limit of detection was lower for Hwp1.2 (dilution 1/2000) compared to Hwp1.1 and 1.3 (1/5000). This lowest sensitivity of Hwp1.2 could explain the lowest diagnostic performances of this fragment.

The above data support that the use of Hwp1.3 as a test protein in an immunoassay of the invention, alone or in combination with some candida cell surface protein of the invention (combination lead to diagnostic performances above the average) show similar or better diagnostic performances (sensibility) than those obtained with actual commercial tests including Mannan/anti-mannan (43-65%) (Serion™ or Platelia ™) or β-glucan assays (77-82%) (Fungitell™)) The use of Hwp1.3 as a test protein in a lateral flow immunoassay of the invention performs much better in terms of sensitivity than the fragment used in the ELISA assay described in WO 2014/001574 as having high sensitivity (88.9% decreased to 33.3% when used in a later flow).

Therefore, a lateral flow immunoassay of the invention has the main advantages of the rapidity of detection (10-15 minutes compared to 2-3 days for existing ELISA) and the predictivity, allowing the detection of the infection before the systemic bloodstream infection stage (e.g. 5-6 days before the detection of the pathogen in blood) which is very promising for the development of a commercial assay which responds to the current needs in the field for a point of care test allowing to get the right information to quickly start an appropriate treatment to reduce the mortality and further contamination risks.

### List of elements referenced in the figures

1 Lateral flow immunoassay
   2 backing support
   3 capillary flow array
      5 sample receiving pad
      6 conjugate release pad
         61 detection reagent
            611a first binder to antibody analyte
            611b first label moiety
         62 detection reagent support
      7 Detection pad
         71 capture test reagent array
            711a test protein (Hwp1.3)
            711b optional further test protein (e.g. Bgl2 or Fba1 or Pgk1 or Set1 or Cfl91
         72 capture control reagent array
            721a control agent specific to first binder (e.g. species-specific antibody for the detection reagent)
         73 detection membrane
   4 wicking pad

### SEQUENCE LISTING

**SEQ ID NO: 1 -Hwp1.3 (synthetic fragment 40-187 from *Candida albicans* Hwp1)** 
SYDYYQEPCDDYPQQQQQEPCDYPQQQQEEPCDYPQQPQEPCDYPQQPQEPCD
YPQQPQEPCDYPQQPQEPCDNPPQPDVPCDNPPQPDVPCDNPPQPDVPCDNPPQPDVP
CDNPPQPDQPDDNPPIPNIPTDWIPNIPTDWIPD.
**SEQ ID NO: 2- comparative Hwp1.2 (synthetic fragment 41-200 from *Candida albicans* Hwp1)** 
YDYYQEPCDDYPQQQQQEPCDYPQQQQEEPCDYPQQQPQEPCDYPQQPQEPCDY
PQQPQEPCDYPQQPQEPCDNPPQPDVPCDNPPQPDVPCDNPPQPDVPCDNPPQPDVPC
DNPPQPDQPDDNPPIPNIPTDWIPNIPTDWIPDIPEKPTTPATTPN.
**SEQ ID NO: 3- comparative Hwp1.1 (synthetic fragment 27-203 from *Candida albicans* Hwp1)** 
GQGETEEALIQKRSYDYYQEPCDDYPQQQQQEPCDYPQQQQEEPCDYPQQQPQE
PCDYPQQPQEPCDYPQQPQEPCDYPQQPQEPCDNPPQPDVPCDNPPQPDVPCDNPPQP
DVPCDNPPQPDVPCDNPPQPDQPDDNPPIPNIPTDWIPNIPTDWIPDIPEKPTTPATTPNI
PA.
**SEQ ID NO: 4 - Eno (*C.albicans* Enolase)** 







.
**SEQ ID NO: 5-Bgl2 (*C.albicans* Bgl2)** 
MQIKFLTTLATVLTSVAAMGDLAFNLGVKNDDGTCKDVSTFEGDLDFLKSHSKIIKT
YAVSDCNTLQNLGPAAEAEGFQIQLGIWPNDDAHFEAEKEALQNYLPKISVSTIKIFL
VGSEALYREDLTASELASKINEIKDLVKGIKDKNGKSYSSVPVGTVDSWNVLVDGAS
KPAIDAADVVYSNSFSYWQKNSQANASYSLFDDVMQALQTLQTAKGSTDIEFWVGE
TGWPTDGSSYGDSVPSVENAADQWQKGICALRAWGINVAVYEAFDEAWKPDTSGT
SSVEKHWGVWQSDKTLKYSIDCKFN.
**SEQ ID NO: 6- Fba1 ((*C.albicans* Fba1)** 
MAPPAVLSKSGVIYGKDVKDLFDYAQEKGFAIPAINVTSSSTVVAALEAARDNKAPII
LQTSQGGAAYFAGKGVDNKDQAASIAGSIAAAHYIRAIAPTYGIPVVLHTDHCAKKL
LPWFDGMLKADEEFFAKTGTPLFSSHMLDLSEETDDENIATCAKYFERMAKMGQWL
EMEIGITGGEEDGVNNEHVEKDALYTSPETVFAVYESLHKISPNFSIAAAFGNVHGVY
KPGNVQLRPEILGDHQVYAKKQIGTDAKHPLYLVFHGGSGSTQEEFNTAIKNGVVKV
 
NLDTDCQYAYLTGIRDYVTNKIEYLKAPVGNPEGADKPNKKYFDPRVWVREGEKTM
SKRIAEALDIFHTKGQL.
**SEQ ID NO: 7-Pgk1 (synthetic fragment 1-238 from *Candida albicans* Pgk1)** 
MSLSNKLSVKDLDVAGKRVFIRVDFNVPLDGKTITNNQRIVAALPTIKYVEEHKPKYI
VLASHLGRPNGERNDKYSLAPVATELEKLLGQKVTFLNDCVGPEVTKAVENAKDGE
IFLLENLRYHIEEEGSSKDKDGKKVKADPEAVKKFRQELTSLADVYINDAFGTAHRA
HSSMVGLEVPQRAAGFLMSKELEYFAKALENPERPFLAILGGAKVSDKIQLIDNLLDK
VDMLIVGGG.
**SEQ ID NO: 8- Set1 (synthetic fragment 1-208 from *Candida albicans* Set1)** 
MSYNNRSGGGASGGYSRRGYHGSHRGGYRTGRSKYPEDRYLVGGMLSLNKGSHYE
SSDNRYIPNEIGSKSPENRSHRSSTKDGRTPSGLSTPLSSSDKVSTPISIESINGSDRNTG
VNNKDSEFPKLSHHSDFTSTIPFSRSINPQKNFMVINDSHTPKTDKGIQSKKIRYNGEG
VNHVSDPRIAQSNSNLQKPTKKTKKTPYKQLPQ.
**SEQ ID NO: 9- Cfl91 (*Candida albicans* Cfl91)** 
MKLLPLSFSFLLFTFTFAKQLPVFDGKTYQSYSHSRNVYACRMHIHKTIKECSTTDIED
YGCMCTNKDAISTFIQCYAYFQKNSTQAIDFFVKYCDNYGNITTNIDQITDAYEDFLN
NGSDTTTITNINNNNNTTNNTTSNHLDFDYSKYAYGLEASIDQFLTNWQRSFFYGMA
MSSYWGFVLLFGTISNWTQYLFPGLMTDLTGPFSNWWRKNLSLPATFGTKKTQEYT
FWFKYLQGYIPSRLESIINSGFCLVVIIVLSINTYYSVGNEIIFTNYVAQLRYLANRTGIN
STILMPFLVLFAGRNNILQWITRWNYGVFMMFHRFIGRMIFLFVVLHAVTFSIALGVD
YYPLMSFPFMIWGTISTVAGGIIMVQASLYVRRRWYEVFLGLHILLALFFMIGGWVHI
SYLGYEWWVFVAIGLWGIDRIVRVLRMISFGFPEADITLMSDETLKIKIPRPDYWEPIP
GGHIFITFLRKDCFWQSHPFTFAPSVENSNTIVLYTKVKQGVTRKLYNYLVTCPGRRA
KITVGVEGPYGECCAAGKSQTAVFIAGGNGVPGMFSEIYDLAKRSTPDSQKRLKLYW
TIREYRSIHWLYEELNELKKFHNVEVTVFVTKPDSNTYINELSSRVISGKCNSTENFDG
ATTATTSLYEKASFDDKATIVVTNRDITSKYDPNSVKDAIIAQLSHVHFREGRPKINTII
KEEIEDAQGSICFVTCGHPAMVDDIRAEVVGTIGKYKEKRIDYYEQLIGWA.
**SEQ ID NO: 10- Hwp1.3 fragment 1 (synthetic)**
   QEPCDYPQQPQEPCDYPQQP.
**SEQ ID NO: 11- Eno fragment 1 (synthetic)**
   DSRGNPTVEVDFTT
**SEQ ID NO: 12- Fba1 fragment 1 (synthetic)**
   YGKDVKDLFDYAQE
**SEQ ID NO: 13- Pgk1 fragment 1 (synthetic)**
   VPLDGKTITNNQRI

## Claims

1. A lateral flow immunoassay device for qualitative or quantitative detection of Candida infection in a sample selected from a whole blood, serum, plasma and a bronchoalveolar lavage (BAL) sample comprising a backing support and, on said backing support, a capillary flow array and a wicking pad, wherein the capillary flow array comprises **i)** a sample receiving pad located at one end of the backing support and having pores so as to receive the sample; **ii)** a conjugate release pad being distinct from the sample receiving pad or included to the sample receiving pad and being in capillary contact with the sample receiving pad and being impregnated with at least one detection reagent comprising a first binder specific and binding to the anti-Hwp1 antibody analyte conjugated to a first label moiety; **iii)** a detection pad being distinct from the conjugate release pad and being in capillary contact with the conjugate release pad and comprising a detection membrane, a capture test reagent array comprising at least one reagent test line and a capture control reagent array comprising one control reagent line, said capture reagent and control reagent arrays being immobilized on the detection membrane and **iv)** a wicking membrane at the other end of the backing support and being in capillary contact with the detection pad, wherein said at least one reagent test line comprises a Hwp1.3 protein and said control reagent line comprises a control reagent having a specific affinity for the first binder.

2. A lateral flow immunoassay device according to claim 1 wherein the conjugate release pad comprises a detection reagent support wherein the said at least said one detection reagent is bound in a capillary releasable manner, receives the sample from the sample receiving pad and releases the detection reagent from its detection reagent support and a first immuno-complex is formed when the anti-Hwp 1 antibody analyte is present in the sample and is combined with the detection reagent, the said first immuno-complex then migrates to the detection pad where it binds to the said at least one reagent test line comprising the Hwp1.3 protein.

3. A lateral flow device according to claim 1 or 2 wherein the detection pad further comprises more than one reagent test line in the capture test reagent array.

4. A lateral flow device according to any one of claims 1 to 3 wherein the capture test reagent array further comprises at least one Candida cell surface protein, for example selected from Eno, Bgl2, Fba1, Pgk1, Set1 and Cfl91 or a combination thereof.

5. A lateral flow immunoassay according to any one of claims 1 to 4, wherein the detection reagent support of the conjugate release pad is non-woven glass fiber.

6. A lateral flow immunoassay according to any one of claims 1 to 5, wherein the detection reagent is an anti-human antibody such as rabbit or mouse anti-human IgG (or IgA) (first binder specific and binding to the antibody analyte) conjugated to colored or fluorescent particles (first label moiety).

7. A lateral flow immunoassay according to any one of claims 1 to 6, wherein the label moiety is selected from a gold nanoparticle, a latex or cellulose particle, a fluorescent label and other colloidal metals and magnetic particles.

8. A lateral flow immunoassay according to any one of claims 1 to 7, wherein the detection membrane is a membrane of nitrocellulose membrane or cellulose.

9. A lateral flow immunoassay according to any one of claims 1 to 8, wherein the capture reagents are immobilized across the detection membrane in two capture reagent arrays (test and control reagents), respectively and each of the capture reagent arrays comprise one or more lines.

10. A lateral flow immunoassay according to any one of claims 1 to 9, wherein it is inserted into a rigid housing (e.g. a plastic cassette) protecting the capillary flow array and wicking pad and having an opening over the sample receiving pad for loading the sample on to said sample receiving pad and one opening over the capture reagent array in order to read and interpret the result of the test.

11. A method a method for measuring the content of anti-Hwpl antibodies in a sample, said method comprising:
a) Providing a sample selected from whole blood, serum, plasma and bronchoalveolar lavage (BAL) potentially contaminated with Candida spp pure or diluted in aqueous solution;
b) Subjecting the said sample to a lateral flow immunoassay comprising **i)** a conjugate release pad comprising detection reagents for said antibodies and **ii)** a detection pad comprising a detection membrane where at least Hwp1.3 protein is bound;
c) Detecting the presence or absence of a detection line on the detection membrane at the location where the said least Hwp1.3 protein is bound, the presence of said detection line being indicative of the presence of anti-Hwp1 antibodies.

12. A method according to claim 11 wherein at least one Candida cell surface protein selected from Eno, Bgl2, Fba1, Pgk1, Set1 and Cfl91 or a combination thereof is further bound to the detection membrane of said lateral flow immunoassay and the detection of the presence of IgG (or IgA) antibodies to this said at least one Candida cell surface protein is further carried out under step c).

13. A method according to claim 11 wherein at least one Candida cell surface protein selected from Eno, Fba1 and Bgl2 is further bound to the detection membrane of said lateral flow immunoassay and the detection of the presence of IgG (or IgA) antibodies to this said at least one Candida cell surface protein is further carried out under step c).

14. A kit for qualitative or quantitative detection of Candida infection in a sample, said kit comprising at least one lateral flow immunoassay device according to any one of claims 1 to 10.

15. A kit according to claim 14 further comprising at least one of the following:
- a blood collection device;
- instructions for use;
- a disinfection pad;
- an adhesive plaster;
- a data reader for reading the assay results and optionally transmitting to a database.
